# EUROPEAN PATENT SPECIFICATION

(11) **EP 2 373 288 B1**
(45) Date of publication and mention of the grant of the patent: **08.04.2015**
(21) Application number: 09799730.8
(22) Date of filing: 16.12.2009
(51) Int. Cl.: A61K 9/06, A61K 31/575, A61K 9/00

(54) **A NOVEL DERMACEUTICAL CREAM MADE USING SODIUM FUSIDATE**
NEUE DERMAZEUTISCHE CREME HERGESTELLT UNTER VERWENDUNG VON FUSIDINSÄURE-NATRIUM
COMPOSITION DERMACEUTIQUE PRÉPAREÉ EN UTILISANT FUSIDATE DE SODIUM

(30) Priority: 19.12.2008 IN MU26452008
(43) Date of publication of application: 12.10.2011
(73) Proprietor: Sulur, Vanangamudi Subramaniam, India, Chennai 600 032 (IN)
(72) Inventor: SRINIVASAN, Madhavan, Chennai 600 032 (IN); CHULLIEL, Neelakandan Narayanan, Chennai 600 032 (IN); KUPPUSAMY, Senthil Kumar, Chennai 600 032 (IN)
(74) Representative: Boult Wade Tennant
(86) International application number: PCT/IB2009/055775
(87) International publication number: WO 2010/070589

(56) References cited:
- WO-A1-2007/087806
- WO-A1-2008/094002
- WO-A1-2010/106458
- ANONYMOUS: "Fucicort-Creme" INTERNET ARTICLE, [Online] 14 October 2004 (2004-10-14), XP002582673 Retrieved from the Internet: URL:HTTP://WWW.PHARMAZIE.COM/GRAPHIC/A/64/ 1-25564.PDF> [retrieved on 2010-05-18]
- Aacis Arzneimittel GmbH: "Fusidinsäure acis Creme"[Online] 1 December 2007 (2007-12-01), pages 1-5, XP002582674 Gebrauchsinformation: Information für den Anwender Retrieved from the Internet: URL:http://gripsdb.dimdi.de/amispb/doc/200 8/03/11/2167931/OBFM0952AF9E01C88004.rtf> [retrieved on 2010-05-18]
- ABDA-BUNDESVEREINIGUNG DEUTSCHER APOTHEKERVERBÄNDE: 'Rezepturhinweise: Fusidinsäure', [Online] pages 1 - 4, XP002582786 PHARMAZEUTISCHES LABORATORIUM, GOVI-VERLAG PHARMAZEUTISCHER VERLAG GMBH Retrieved from the Internet: <URL:http://www.pharmazeutische-zeitung.de/ fileadmin/nrf/PDF/1-Fusidinsaeure.pdf> [retrieved on 2010-05-18]

## Description

### Field Of Invention

The present invention relates to primary and secondary bacterial skin infections and in particular it relates to the treatment of these infections using a Fusidic acid cream that has been made using Sodium Fusidate as the starting Active Pharmaceutical Ingredient (API).

### Background Of The Invention

Numerous treatments, both topical and systemic, are available for the primary and secondary skin infection caused by sensitive Gram +ve organisms such as Staphylococcus aureus, Streptococcus spp etc. Topical and systemic bacterial infection treatment compositions typically employ at least one active pharmaceutical ingredient (API) in combination with a base component. In the cream form, the APIs typically comprise an antibiotic/antibacterial such as Fusidic acid and the like.

In the currently available Fusidic acid creams, Fusidic acid in fine powder form is used as source API. The small particle size enhances its dermal contact by providing a large specific surface area and penetration, and provides a smooth feel on application to skin. However, a serious shortcoming of the fine size of Fusidic acid particles is that it presents an enormous surface area for contact and reaction with molecular Oxygen during manufacture, handling, and processing of the cream. This has serious implications to its chemical stability and results in rapid reduction in potency of the API (Fusidic acid) in the final cream formulation. Degradation due to oxidation is a major cause of instability of currently available Fusidic acid creams. Table 1 show that the degradation in the API samples (Fusidic acid) exposed to oxygen ranged between 7.7% and 11% for conditions ranging from room temperature to 45 °C when analysed at three months of exposure period at the above conditions.

It is known that greater the exposure time of Fusidic acid as the raw API to Oxygen, greater the limitations on stabilising Fusidic acid in a formulation. However, there is no published data on the stability of Fusidic acid over a period of time.

As an alternative to Fusidic acid, Sodium Fusidate is known to have been used to make dermaceutical medicaments for topical application. However, these are in the form of ointment rather than cream. Drawbacks of ointments over creams are well known and it's generally preferable to use creams rather than ointments for topical application.

Several aspects of Fusidic acid as an API are known:
- It is thermolabile
- It is available in cream formulations
- It can be obtained from Sodium Fusidate by dissolving the latter in an aqueous phase and adding acid to the solution, whereby Fusidic acid precipitates. However, the Fusidic acid precipitate is difficult to process into a cream form first due to its coarse and uneven particle size and second retrieving Fusidic acid from wet cake involves drying and further handling which deteriorates the Fusidic acid due to exposure to oxygen
- The stability of the API in a Fusidic acid cream is unreliable due to the thermolabile nature of Fusidic acid

Stabilization of medicaments containing Fusidic acid against oxidation involves observing a number of stringent precautionary procedures during manufacture and storage. These include:
- replacing Oxygen in pharmaceutical containers with inert gases such as Nitrogen, Carbon dioxide, Helium and the like
- avoiding contact of the medicament with heavy metal ions which catalyze oxidation,
- storing the API at reduced temperatures throughout its shelf life before processing

In practice this means stricter controls during the manufacture as well as storage of such API (storing it typically at 2°C to 8°C in air-tight containers throughout their shelf life).

There is therefore a need to provide a Fusidic acid cream in which Fusidic acid will be of greater stability at the time of the manufacture of the cream, and which will sustain its stability at an acceptable level throughout its shelf life.

### Objects And Advantages Of The Invention

It is therefore one object of the present invention to provide a cream which contains Fusidic acid as the active API but which has greater stability of the API throughout its shelf life.

### Brief Summary Of The Invention

The invention discloses a dermaceutical cream containing Fusidic acid which is formed in situ from Sodium Fusidate as the starting raw material, wherein Sodium Fusidate is converted into Fusidic acid under oxygen-free environment. The cream of the present invention has greater shelf-life stability and the finer particle size of the API than the conventional creams containing Fusidic acid. The cream of the present invention contains Fusidic acid as the API that has been formed in situ from Sodium Fusidate, in a cream base comprising an acid, a co-solvent, an emulsifier and a waxy material along with water, preferably purified water.

### Detailed Description Of The Invention

We discussed earlier the known aspects of the topical preparations that have Fusidic acid and Sodium Fusidate as the APIs. It is evident from the current state of knowledge that:
- Creams containing Fusidic acid that are made using Sodium Fusidate as starting API are not available.
- There is no published data on the stability of Sodium Fusidate as the API.
- Sodium Fusidate is not considered to be inherently more stable as an API than Fusidic acid.

In the face of this, it has been surprisingly discovered that Sodium Fusidate as an API is significantly more stable than Fusidic acid and that Fusidic acid deteriorates more rapidly than Sodium Fusidate.

There is no published data on the stability of Sodium Fusidate as the API. The applicant carried out experiments on Sodium Fusidate to evaluate its stability. It can be seen from Table 2 that the degradation of Sodium Fusidate over a temperature range of room temperature to 45 °C ranged between 2.45 % and 6%.

Tables 1 and 2 also show the comparison between the stability of the Fusidic acid and Sodium Fusidate as raw APIs. The study was carried out using an in-house HPLC method developed by the applicant, which the applicant believes is a true stability-indicating method as opposed to the titration method suggested in British Pharmacopoeia (BP). This is because the BP method does not differentiate between the intact API and the degraded form.

### Stability Analysis of Fusidic Acid:

**Table 1: Results Of 3 Months Old Fusidic Acid (API) Analysis By Stability Indicating HPLC Method And Titration Method**

| S.No | Conditions | *Initial (%) | Fusidic Acid Assay (%) | | Percentage Drop (%) | | Remarks |
|---|---|---|---|---|---|---|---|
| | | | Titration | HPLC | Titration | HPLC | |
| 1 | RT (Open) | 100.6 | 99.21 | 92.93 | 1.39 | 7.67 | API analysed After 3 Months |
| 2 | RT (Closed) | | 99.02 | 94.37 | 1.58 | 6.23 | |
| 3 | 45°C (Open) | | 98.52 | 89.52 | 2.08 | 11.08 | |
| 4 | 45°C (Closed) | | 99.10 | 92.12 | 1.50 | 8.48 | |

| | | | | | | | |
|---|---|---|---|---|---|---|---|
| Name of the Sample: **FUSIDIC ACID BP** Pack : Open & Closed Petri dish | | | | | | | |

### Stability Analysis of Sodium Fusidate:

**Table 2 : Results Of 3 Months Old Sodium Fusidate (API) Analysis By Stability Indicating HPLC Method And Titration Method**

| S.No | Conditions | *Initial (%) | Sodium Fusidate Assay(%) | | Percentage (%) | | Remarks |
|---|---|---|---|---|---|---|---|
| | | | Titration | HPLC | Titration | HPLC | |
| 1 | RT (Open) | 98.7 | 97.71 | 96.25 | 0.99 | 2.45 | API analysed3 After Months |
| 2 | RT (Closed) | | 98.85 | 97.67 | -0.15 | 1.03 | |
| 3 | 45°C (Open) | | 97.07 | 92.65 | 1.63 | 6.05 | |
| 4 | 45°C (Closed) | | 97.16 | 92.96 | 1.54 | 5.74 | |

| | | | | | | | |
|---|---|---|---|---|---|---|---|
| Name of the Sample: **Sodium Fusidate BP** Pack: Open & Closed Petri dish | | | | | | | |

In both studies the * Initial denotes the results of the samples tested at the time of receipt of the API from the supplier.

It can be observed from Tables 1 and 2 that:
- In the case of Fusidic Acid, there is about 7.7% loss in 3 Months at room temperature (open condition) and about 11% loss in 3 Months at 45°C (open condition).
- In the case of Sodium Fusidate, there is about 2.5% loss in 3 Months at room temperature (open condition) and about 6% loss in 3 Months at 45°C (open condition).

The data thus shows that Sodium Fusidate as an API is more stable than Fusidic acid.

The applicants explored the possibility of making a cream (rather than an ointment) using Sodium Fusidate (rather than Fusidic acid). Although Sodium Fusidate has been used in dermaceutical applications, it has not been possible to make creams that use Sodium Fusidate. This is because of the inherent alkalinity of Sodium Fusidate (pH 7.5 to 9), which means it cannot be used in a cream form therefore all products manufactured using Sodium Fusidate as starting material are ointments. A dermaceutical cream that uses Sodium Fusidate would exploit the benefit of the fact that Sodium Fusidate is more stable than Fusidic acid and it would also provide a cream formulation which is far superior in its application qualities than an ointment. It would thus fill an existing need for a cream that has better stability than currently available creams containing Fusidic acid.

The applicant therefore surprisingly discovered that in order to achieve greater stability of the API in a dermaceutical cream, Sodium Fusidate rather than Fusidic acid may be used as the starting API during the cream's manufacture. Using Sodium Fusidate as starting material eliminates the drawback associated with the manufacture and storage of existing Fusidic acid creams.

The applicant has also discovered that the Fusidic acid cream prepared using Sodium Fusidate as the staring API shows good chemical stability, efficacy, and microbial sensitivity.

The application discloses a cream containing Fusidic acid (the API) that has been prepared using Sodium Fusidate as the starting API, in which Fusidic acid forms in-situ under totally oxygen free environment by slow addition of an acid, into a molecular dispersion form (due to the presence of a co-solvent) at the intermediate stage, and which Fusidic acid regenerates as an extremely fine dispersion when added to a final cream base, thereby resulting in a finely and homogeneously dispersed Fusidic acid in the final cream. All these operations are performed in an environment free of atmospheric oxygen. The cream of the present invention contains Fusidic acid as the API that has been formed in situ from Sodium Fusidate, in a cream base comprising an acid, a co-solvent, an emulsifier and a waxy material along with water, preferably purified water.

The APIs which may be employed in the present invention as starting APIs are either acid-based actives or their salts well known in the art of treating bacterial primary and secondary infections. Examples of suitable acid-based actives or their salts which may be used include, but are not limited to Sodium Fusidate.

These acid-based active compounds or their salts require a base component to be used in the pharmaceutical composition that uses the compounds, since the compounds cannot, by themselves, be deposited directly on to human skin due to their harshness.

The cream base of the present invention optionally further comprises an ingredient selected from a group comprising a preservative, a buffering agent, an anti oxidant, a chelating agent, and a humectant, or any combination thereof.

The present invention provides a novel cream that has been produced using Sodium Fusidate as the starting raw material, and which cream contains Fusidic acid of high therapeutic efficacy and of chemical stability that is generally superior to the commercially available creams containing Fusidic acid.

The Fusidic acid cream of the present invention has been manufactured in a totally oxygen free environment under purging with inert gas and applying vacuum. Under these conditions, the Sodium Fusidate is converted in situ into Fusidic acid. The cream of the present invention is used in the treatment of bacterial skin infections.

The pH of the product of the present invention is from about 3 to 6. On the other hand, Sodium Fusidate ointments that are commercially available are greasy and cosmetically non elegant.

It is essential that the active drug penetrates the skin for the optimum bio-dermal efficacy. The particle size of the active drug plays an important role here. It is necessary that the active drug is available in a finely dispersed form for the product to be being efficacious. Also this is to be achieved in the safe pH compatible environment of skin (4.0 to 6.0). To achieve all these, it is essential to choose proper vehicles or co-solvents for the dissolution or dispersion of the drug.

Particle size analysis was carried out on the present invention (Apex product) and on some commercially available product samples (samples A, C, D, F, G, and K). Maximum and minimum particle sizes, mean particle size and standard deviation and the coefficient of variation were assessed. Table 3 shows a comparison.

**Table 3**

| | **Minimum Particle Size (µm)** | **Maximum Particle Size (µm)** | **Mean Particle Size (µm)** | **Standard Deviation** | **Coefficient of Variation** |
|---|---|---|---|---|---|
| Present Invention (Apex) | 2.33 | 16.30 | 10.01 | 3.982 | 0.397 |
| A | 7.23 | 39.58 | 18.09 | 9.251 | 0.511 |
| C | 6.07 | 32.69 | 14.11 | 6.692 | 0.474 |
| D | 9.8 | 27.52 | 18.48 | 4.98 | 0.269 |
| F | 7.93 | 19.90 | 14.82 | 4.033 | 0.272 |
| G | 7.29 | 29.48 | 15.25 | 6.065 | 0.398 |
| K | 5.75 | 32.63 | 16.80 | 8.112 | 0.483 |

The particle size distribution analysis clearly indicates the presence of Fusidic acid of fine particle size in the product of the present invention, the size that is much reduced than the conventional products. This is attributed to the fact that the instant product is made using Sodium Fusidate using in situ conversion of Sodium Fusidate to Fusidic acid in a finely dispersed form. All of the measured parameters are better than those found for the commercially available creams containing Fusidic acid. This is another clear advantage of the product disclosed herein over the commercially available products.

The product of the present invention is efficacious due to the pronounced antibacterial activity of the regenerated Fusidic acid which is available in reduced particle size than the conventional products, and in a finely dispersed form.

The inventor has screened different co-solvents such as Propylene Glycol, Hexylene Glycol, PolyEthyleneGlycol-400 & the like and dissolved the Sodium Fusidate in one of above co-solvents varying from about 5% (w/w) to 40% (w/w) under inert gas purging and under vacuum and converted to Fusidic acid in-situ by adding an acid such as HCl, H₂SO₄, HNO₃, Lactic acid and the like from about 0.005% (w/w) to about 0.5% (w/w) under stirring and obtained Fusidic acid in more stabilized and solution form, which makes our final product in a cream base which easily penetrates the skin and highly efficacious, and also highly derma compatible by having a pH of about 3.0 to about 6.0.

The stability of the product is confirmed by the stability studies performed for 6 months as per ICH guidelines and a comparison of stress studies done for in-house product with those on samples of commercially available comparable products.

### Experimental Data

API-stability experiments were carried out (see tables 4-14) using the product of the present invention and products currently commercially available. Tests were carried out to observe (or measure as appropriate) the physical appearance of the product, the pH value and assay of the API over a period of time. Tests were also carried out to assess the stability by subjecting the product to stress studies such as autoclave test and oxydative degradation test. Further, in vitro antimicrobial zone of inhibition studies were also carried out over a period of time. Each gram of product of the present invention used for the tests contained Sodium Fusidate in the amount required to produce 2% (w/w) Fusidic acid in the finished product.

The product used for the Stability Studies, Autoclave and Oxydative degradation tests contained approximately 10% extra API (overages). The product of the present invention used for studies contained Fusidic acid cream prepared using Sodium Fusidate as starting material. It was packaged in an aluminium collapsible tube and each gram of the product contained 20.8 mg of Sodium Fusidate (in conformance with BP), which is equivalent to 20 mg of Fusidic acid (BP conformant). The details of the analyses on commercially available comparable products (Fusidic Acid creams) are provided in the tables 13-A and 14 as appropriate.

**Table 4: Description Test, Batch No. ASF-09**

| **Measured parameter:** Physical appearance | | | | | |
|---|---|---|---|---|---|
| **Best possible value of measured parameter:** Homogeneous White to off White Viscous cream **Method of measurement:** Observation by naked eye | | | | | |
| Conditions | **Initial** | **1^{st} Month** | **2^{nd} Month** | **3^{rd} Month** | **6^{th} Month** |
| 40°C 75% RH | Homogenous White to off White viscous cream | Best possible value | Best possible value | Best possible value | Best possible value |
| 30°C 65% RH | | Do | Do | Do | Do |
| 25°C 60% RH | | Do | Do | Do | Do |
| Temperature cycling | | Do | - | - | - |
| Freezthaw | | Do | - | - | - |

**Table 5: pH Test, Batch No. ASF-09**

| **Measured parameter:** pH | | | | | |
|---|---|---|---|---|---|
| **Limits of measured parameter:** 3-6 | | | | | |
| **Method of measurement:** Digital pH Meter | | | | | |
| Conditions | **Initial** | **1^{st} Month** | **2^{nd} Month** | **3^{rd} Month** | **6^{th} Month** |
| 40°C 75% RH | 4.22 | 4.21 | 4.22 | 4.20 | 4.19 |
| 30°C 65% RH | | 4.20 | 4.21 | 4.21 | 4.20 |
| 25°C 60% RH | | 4.21 | 4.21 | 4.20 | 4.19 |
| Temperature cycling | | 4.22 | - | - | - |
| Freezthaw | | 4.21 | - | - | - |

**Table 6: Assay (%) Test, Batch No. ASF-09**

| **Measured parameter:** Assay (%) | | | | | |
|---|---|---|---|---|---|
| **Limits of measured parameter:** 90-110% | | | | | |
| **Method of measurement:** HPLC Method | | | | | |
| Conditions | **Initial** | **1^{st} Month** | **2^{nd} Month** | **3^{rd} Month** | **6^{th} Month** |
| 40°C 75% RH | 108.60 | 108.56 | 108.26 | 108.11 | 108.05 |
| 30°C 65% RH | | 108.53 | 108.36 | 108.26 | 108.11 |
| 25°C 60% RH | | 108.59 | 108.45 | 108.39 | 108.26 |
| Temperature cycling | | 107.53 | - | - | - |
| Freezthaw | | 108.01 | - | - | - |

**Table 7: Description Test, Batch No. ASF-10**

| **Measured parameter:** Physical appearance | | | | | |
|---|---|---|---|---|---|
| **Best possible value of measured parameter:** Homogeneous White to off White Viscous cream | | | | | |
| **Method of measurement:** Observation by naked eye: | | | | | |
| Conditions | **Initial** | **1^{st} Month** | **2^{nd} Month** | **3^{rd} Month** | **6^{th} Month** |
| 40°C 75% RH | Homogenous White to off White viscous cream | Homogenous White to off White viscous cream | Homogenous White to off White viscous cream | Homogenous White to off White viscous cream | Homogenous White to off White viscous cream |
| 30°C 65% RH | | -do- | -do- | -do- | -do- |
| 25°C 60% RH | | -do- | -do- | -do- | -do- |
| Temperature cycling | | -do- | - | - | - |
| Freezthaw | | -do- | - | - | - |

**Table 8: pH Test, Batch No. ASF-10**

| **Measured parameter:** pH | | | | | |
|---|---|---|---|---|---|
| **Limits of measured parameter:** 3-6 | | | | | |
| **Method of measurement:** Digital pH Meter | | | | | |
| Conditions | **Initial** | **1^{st} Month** | **2^{nd} Month** | **3^{rd} Month** | **6^{th} Month** |
| 40°C 75% RH | 4.23 | 4.22 | 4.21 | 4.20 | 4.20 |
| 30°C 65% RH | | 4.21 | 4.20 | 4.21 | 4.21 |
| 25°C 60% RH | | 4.20 | 4.21 | 4.21 | 4.20 |
| Temperature cycling | | 4.21 | - | - | - |
| Freezthaw | | 4.20 | - | - | - |

**Table 9: Assay (%) Test, Batch No. ASF-10**

| **Measured parameter:** Assay (%) | | | | | |
|---|---|---|---|---|---|
| **Limits of measured parameter:** 90-110% | | | | | |
| **Method of measurement:** HPLC Method | | | | | |
| Conditions | **Initial** | **1^{st} Month** | **2^{nd} Month** | **3^{rd} Month** | **6^{th} Month** |
| 40°C 75% RH | 108.50 | 108.46 | 108.36 | 108.15 | 108.04 |
| 30°C 65% RH | | 108.43 | 108.29 | 108.22 | 108.10 |
| 25°C 60% RH | | 108.49 | 108.45 | 108.41 | 108.34 |
| Temperature cycling | | 107.43 | - | - | - |
| Freezthaw | | 108.03 | - | - | - |

**Table 10: Description Test, Batch No. ASF-12**

| **Measured parameter:** Physical appearance | | | | | |
|---|---|---|---|---|---|
| **Best possible value of measured parameter:** Homogeneous White to off White Viscous cream | | | | | |
| **Method of measurement:** Observation by naked eye | | | | | |
| Conditions | **Initial** | **1^{st} Month** | **2^{nd} Month** | **3^{rd} Month** | **6^{th} Month** |
| 40°C 75% RH | Homogenous White to off White viscous cream | Homogenous White to off White viscous cream | Homogenous White to off White viscous cream | Homogenous White to off White viscous cream | Homogenous White to off White viscous cream |
| 30°C 65% RH | | -do- | -do- | -do- | -do- |
| 25°C 60% RH | | -do- | -do- | -do- | -do- |
| Temperature cycling | | -do- | - | - | - |
| Freezthaw | | -do- | - | - | - |

**Table 11: pH Test, Batch No. ASF-12**

| **Measured parameter:** pH | | | | | |
|---|---|---|---|---|---|
| **Limits of measured parameter:** 3-6 | | | | | |
| **Method of measurement:** Digital pH Meter | | | | | |
| Conditions | **Initial** | **1^{st} month** | **2^{nd} month** | **3^{rd} Month** | **6^{th} Month** |
| 40°C 75% RH | 4.24 | 4.23 | 4.22 | 4.22 | 4.23 |
| 30°C 65% RH | | 4.24 | 4.23 | 4.23 | 4.22 |
| 25°C 60% RH | | 4.23 | 4.22 | 4.22 | 4.21 |
| Temperature cycling | | 4.22 | - | - | - |
| Freezthaw | | 4.23 | - | - | - |

**Table 12: Assay (%) Test, Batch No. ASF-12**

| **Measured parameter:** Assay (%) | | | | | |
|---|---|---|---|---|---|
| **Limits of measured parameter:** 90-110% | | | | | |
| **Method of measurement:** HPLC Method | | | | | |
| Conditions | **Initial** | **1^{st} Month** | **2^{nd} month** | **3^{rd} Month** | **6^{th} Month** |
| 40°C 75% RH | 108.59 | 108.44 | 108.41 | 108.36 | 108.10 |
| 30°C 65% RH | | 108.41 | 108.40 | 108.32 | 108.15 |
| 25°C 60% RH | | 108.42 | 108.36 | 108.31 | 108.28 |
| Temperature cycling | | 107.64 | - | - | - |
| Freezthaw | | 108.11 | - | - | - |

It is apparent from the review of tables 4-12 that on all counts, the pH value, the physical appearance, and stability, the product of the present invention is quite good.

Table 13 provides reference dates for samples A-I which were taken from commercially available creams of Fusidic acid and used for analyses.

**Table 13**

| **Sample Number** | **Mfg. Date** | **Exp. Date** |
|---|---|---|
| Present invention (apex) | Oct'09 | Sep'11 |
| Sample A | Aug'09 | Jul'11 |
| Sample B | Aug'09 | Jul'11 |
| Sample C | Jul'09 | Jun'11 |
| Sample D | Jul'09 | Jut'11 |
| Sample E | Aug'09 | Jul'11 |
| Sample F | Aug'09 | Jul'11 |
| Sample G | Aug'09 | Jul'11 |
| Sample H | Jul'09 | Jun'11 |
| Sample I | Dec'09 | Nov-11 |

**Table 13-A: Autoclave Analysis (%) Test,**

| **Measured parameter:** Assay (%) | | | | | | | | |
|---|---|---|---|---|---|---|---|---|
| **Limits of measured parameter:** 90-110% | | | | | | | | |
| **Method of measurement:** HPLC Method | | | | | | | | |
| **Sr. No** | **Name of the Products and Details** | **Analysis-I (%)** | | | **Analysis-II (%)** | | | **Average drop of Analysis-1 & in Analysis-II (%)** |
| | | **Initial** | **After Autoclave** | **Drop in %** | **Initial After** | **After Autoclave** | **Drop in %** | |
| 1 | invention (Apex) | 110.47 | 104.61 | 5.86 | 110.62 | 104.86 | 5.76 | **5.81** |
| 2 | Sample A | 101.81 | 91.79 | 10.02 | 100.93 | 91.65 | 9.28 | **9.65** |
| 3 | Sample B | 92.69 | 83.54 | 9.15 | 91.13 | 83.08 | 8.05 | **8.6** |
| 4 | Sample C | 110.47 | 98.56 | 11.91 | 110.2 | 99.21 | 10.99 | **11.45** |
| 5 | Sample D | 101.3 | 94.84 | 6.46 | 102.13 | 94.65 | 7.48 | **6.97** |
| 6 | Sample E | 100.99 | 94.51 | 6.48 | 100.21 | 93.51 | 6.70 | **6.59** |
| 7 | Sample F | 96.33 | 84.15 | 12.18 | 95.88 | 85.12 | 10.76 | **11.47** |
| 8 | Sample G | 104.75 | 93.19 | 11.56 | 103.25 | 93.12 | 10.13 | **10.84** |
| 9 | Sample H | 101.26 | 88.35 | 12.91 | 100.86 | 87.98 | 12.88 | **12.89** |
| 10 | Sample I | 101.58 | 87.06 | 14.52 | 100.61 | 88.01 | 12.6 | **13.56** |

**Table 14: Oxidative degradation Analysis (%) Test,**

| **Measured parameter:** Assay (%) | | | | |
|---|---|---|---|---|
| **Limits of measured parameter:** NA | | | | |
| **Method of measurement:** HPLC Method | | | | |
| **Sr. No** | **Name of the Products and Details** | **Analysis(%)** | | |
| | | **Initial** | **After Oxidation** | **Degradation in %** |
| 1 | invention (Apex) | 110.47 | 106.75 | 3.72 |
| 2 | Sample A | 101.81 | 95.63 | 6.18 |
| 3 | Sample B | 92.69 | 83.15 | 9.54 |
| 4 | Sample C | 110.47 | 101.93 | 8.54 |
| 5 | Sample D | 101.3 | 93.25 | 8.05 |
| 6 | Sample E | 100.99 | 95.47 | 5.52 |
| 7 | Sample F | 96.33 | 90.70 | 5.63 |
| 8 | Sample G | 104.75 | 96.46 | 8.29 |
| 9 | Sample H | 101.26 | 94.53 | 6.73 |
| 10 | Sample I | 101.58 | 88.92 | 12.66 |

**Inference from Table 13-A:** The assay results of Autoclave analysis (121°C applied for 15 Minutes) indicate that the commercially available samples of Fusidic acid cream (Sr. Nos. 2-10) show more percentage drop in API content than for the product of the present invention (Sr. no. 1).

**Inference from Table 14:** The above Assay results of Oxidative degradation analysis (30% Hydrogen peroxide Solution over a period of 12 hours) indicate that the various Market samples of Fusidic acid cream (Sr. Nos. 2-10) show significantly higher API degradation (indicated by the percentage drop in API content) than for the product of the present invention (Sr. no. 1).

From the above data, it is evident that product of the present invention is quite stable at ambient conditions and also at elevated temperature & humid conditions of storage. Also the autoclave studies & Oxidative degradation studies further confirm the stability of the product. This is a major advantage over the currently available Fusidic acid creams. The stability of the product is further ascertained by the shelf-life prediction of the formulation using arrhenius plot of degradation employing Nova-LIMS software.

The antimicrobial/antibacterial activity of the product is confirmed by the in vitro Antimicrobial Zone of Inhibition studies for the product against Staphylococcusaureus. The details of the studies are detailed below in Table 15.

**Table 15**

| **S. No** | **Sample** | **Dose** | **Zone Diameter Range (mm)** | **Inference** |
|---|---|---|---|---|
| 1 | Reference standard (Fusidic acid) | 10mcg | 21 - 33 | Sensitive |
| | | 20mcg | 20 - 30 | Sensitive |
| | | 50mcg | 25 - 32 | Sensitive |
| 2 | Positive control (Penicillin G) | 10 Units | 21 - 27 | Resistant |
| 3 | Negative control (DMSO 1%) | NA | NIL | NIL |
| 4 | Sample (Test Substance) (ASF- product of the present invention 2%) | 10mcg | 21 - 23 | Sensitive |
| | | 20mcg | 24 - 26 | Sensitive |
| | | 50mcg | 21 - 24 | Sensitive |

From the above data it is evident that the product has adequate antimicrobial/antibacterial activity to treat primary and secondary bacterial infections.

According to the preferred embodiment of the present invention, there is provided a composition for the topical treatment of bacterial skin infections on human skin, the composition comprising Fusidic acid made in situ by a conversion of Sodium Fusidate, a cream base containing primary and secondary emulsifiers, waxy materials, co-solvents, and acids, and water.

The proportions of various components of the preferred embodiment are as follows:
a. Fusidic acid from about 0.1% (w/w) to about 25% (w/w) by weight, preferably from about 0.5% (w/w) to about 5%(w/w) by weight and more preferably about 2.00 % (w/w), which has been converted in situ from Sodium Fusidate from about 0.1% (w/w) to about 25% (w/w) by weight, preferably from about 0.5% (w/w) to about 5% (w/w) by weight and more preferably about 2.08% (w/w), and
b. a cream base containing primary and secondary emulsifiers, waxy materials, co-solvents, acids, and water wherein
   - primary and secondary emulsifiers are selected from a group comprising Cetostearyl alcohol, Cetomacrogol-1000, Polysorbate-80, Span-80 and the like from about 1% (w/w) to 15% (w/w), preferably 15% (w/w), more preferably 14.5% (w/w)
   - waxy materials are selected from a group comprising White Soft Paraffin, Liquid Paraffin, Hard Paraffin and the like from about 5% (w/w) to 20% (w/w), preferably 15% (w/w), more preferably 12.5% (w/w),
   - co-solvents are selected from a group comprising Propylene Glycol, Hexylene Glycol, PolyEthylene Glycol-400 and the like from about 5% (w/w) to 40% (w/w), preferably 30% (w/w), more preferably 25% (w/w),
   - acids are selected from a group comprising HCl, H2So4, HNO3, Lactic acid and the like from about 0.005% (w/w) to 0.5% (w/w), preferably 0.3% (w/w), more preferably 0.25% (w/w), and
   - water in the amount in the range of 20% (w/w) to 75% (w/w), preferably 35% (w/w) to 50% (w/w), more preferably 40% (w/w) to 43% (w/w), preferably purified water.

In another embodiment of the present invention the product of the preferred embodiment is further provided with preservatives, wherein said preservatives are selected from a group comprising Methylparaben, Propylparaben, Chlorocresol, Potassium sorbate, Benzoic acid and the like from about 0.05% (w/w) to 0.5% (w/w), preferably 0.3% (w/w), more preferably 0.2% (w/w).

In a still further embodiment of the present invention, the product of the preferred embodiment is further provided with a buffering agent selected from a group comprising Di Sodium Hydrogen Ortho Phosphate, Sodium Hydrogen Ortho Phosphate and the like from about 0.01% (w/w) to 1.00% (w/w), preferably 0.5% (w/w), more preferably 0.05% (w/w).

In yet another embodiment of the present invention, the product of the preferred embodiment is further provided with an anti oxidants are selected from a group comprising Butylated Hydroxy Anisole, Butylated Hydroxy Toluene and the like from about 0.001% (w/w) to 5% (w/w), preferably 0.1% (w/w), more preferably 0.01% (w/w).

In a further embodiment of the present invention, the product of the preferred embodiment is further provided with a chelating selected from a group comprising Disodium EDTA and the like from about 0.01% (w/w) to 1% (w/w), preferably 0.5% (w/w), more preferably 0.1% (w/w).

In still another embodiment of the present invention, the product of the preferred embodiment is further provided with a humectant selected from a group comprising Glycerin, Sorbitol, Propylene glycol and the like from about 5% (w/w) to 40% (w/w) preferably 30% (w/w), more preferably 25% (w/w).

In another embodiment of the present invention, the product of the preferred embodiment further is provided with at least one component selected from a group comprising buffering agents, preservatives, anti oxidants, chelating agents, humectants, or any combination thereof in respective proportions disclosed in the earlier described embodiments.

In a further embodiment of the present invention, a novel dermaceutical cream is disclosed wherein sodium fusidate is converted in-situ under totally oxygen free environment by slow addition of an acid, into Fusidic acid of a molecular dispersion form (due to the presence of a co-solvent) at the intermediate stage, and which Fusidic acid regenerates into an extremely finely dispersed form when added to a final cream base, thereby resulting in a finely and homogeneously dispersed Fusidic acid in the final cream; all operations of converting sodium fusidate into Fusidic acid carried out preferably in an environment free of atmospheric oxygen.

**Table 16: Composition of the typical cream of the preferred embodiment of the present invention**

| **S.No** | **Ingredients** | **Specification** | **% (w/w)** |
|---|---|---|---|
| 1 | Fusidic acid made from Sodium Fusidate | BP | 2.00 |
| 2 | Cetostearyl Alcohol | IP | 12.5 |
| 3 | White Soft Paraffin | IP | 12.5 |
| 4 | Polysorbate 80 | IP | 2 |
| 5 | Propylene Glycol | IP | 25 |
| 6 | Benzoic Acid | IP | 0.2 |
| 7 | Butylated Hydroxy Toluene | IP | 0.01 |
| 8 | Disodium Edetate | IP | 0.1 |
| 9 | 1 M Nitric Acid | IP | 4.0 |
| 10 | Disodium hydrogen Orthophosphate anhydrous | IP | 0.05 |
| 11 | Purified Water | IP | 41.56 |

It is evident from the foregoing description that the present invention comprises the following embodiments.
1. A novel dermaceutical cream containing Fusidic acid which is made in situ under oxygen-free environment using Sodium Fusidate, wherein said cream comprises Fusidic acid made in situ by a conversion of Sodium Fusidate, and a cream base containing at least one of each of a primary and secondary emulsifier,, a waxy material, a co-solvents, an acid, and water, preferably purified water.
2. A novel dermaceutical cream as described in item 1, wherein said Fusidic acid is present in an amount from about 0.1% (w/w) to about 25% (w/w), preferably from about 0.5% (w/w) to about 5%(w/w), and more preferably about 2.00 % (w/w), and in which the amount of said Sodium Fusidate used to form in situ said Fusidic acid is in the range between about 0.1% (w/w) to about 25% (w/w), preferably from about 0.5% (w/w) to about 5% (w/w) and more preferably about 2.08 % (w/w), and
   said primary and secondary emulsifier is selected from a group comprising Cetostearyl alcohol, Cetomacrogol-1000, Polysorbate-80, Span-80 and the like, either singly or any combination thereof, to form a proportion from about 1% (w/w) to 15% (w/w), preferably 15% (w/w), more preferably 14.5% (w/w),
   said waxy material is selected from a group comprising White soft paraffin, Liquid Paraffin, Hard paraffin and the like, either singly or any combination thereof, to form a proportion from about 5% (w/w) to 20% (w/w), preferably 15% (w/w), more preferably 12.5% (w/w),
   said co-solvent is selected from a group comprising Propylene Glycol, Hexylene Glycol, PolyEthylene Glycol-400 and the like, either singly or any combination thereof, to form a proportion from about 5% (w/w) to 40% (w/w), preferably 30% (w/w), more preferably 25% (w/w),
   said acid is selected from a group comprising acids such as HCl, H2So4, HNO3, Lactic acid and the like, either singly or any combination thereof, to form a proportion from about 0.005% (w/w) to 0.5% (w/w), preferably 0.3% (w/w), more preferably 0.25% (w/w), and
   water in the amount in the range of 20% (w/w) to 75% (w/w), preferably 35% (w/w) to 50% (w/w), more preferably 40% (w/w) to 43% (w/w), preferably purified water.
3. A novel dermaceutical cream as described in item 2 which further comprises a preservative, wherein said preservatives is selected from a group comprising Methylparaben, Propylparaben, Chlorocresol, Potassium sorbate, Benzoic acid and the like, either singly or any combination thereof, to form a proportion from about 0.05% (w/w) to 0.5% (w/w), preferably 0.3% (w/w), more preferably 0.2% (w/w).
4. A novel dermaceutical cream as described in items 2-3 which further comprises a buffering agent, wherein said buffering agent is selected from a group comprising Di Sodium Hydrogen Ortho Phosphate, Sodium Hydrogen Ortho Phosphate and the like, either singly or any combination thereof, to form a proportion from about 0.01% (w/w) to 1.00% (w/w), preferably 0.5% (w/w), more preferably 0.05% (w/w).
5. A novel dermaceutical cream as described in items 2-4 which further comprises an anti-oxidant, wherein said anti-oxidant is selected from a group comprising Butylated Hydroxy Anisole, Butylated Hydroxy Toluene and the like, either singly or any combination thereof, to form a proportion from about 0.001% (w/w) to 5% (w/w), preferably 0.1% (w/w), more preferably 0.01% (w/w).
6. A novel dermaceutical cream as described in items 2-5 which further comprises a chelating agent, wherein said chelating agent is selected from a group comprising Disodium EDTA and the like, either singly or any combination thereof, to form a proportion from about 0.01% (w/w) to 1% (w/w), preferably 0.5% (w/w), more preferably 0.1% (w/w).
7. A novel dermaceutical cream as described in items 2-6 which further comprises a humectant, wherein said humectant is selected from a group comprising Glycerin, Sorbitol, Propylene glycol and the like, either singly or any combination thereof, to form a proportion from about 5% (w/w) to 40% (w/w), preferably 30% (w/w), more preferably 25% (w/w).
8. A novel dermaceutical cream as described in items 1-7 wherein sodium fusidate is converted in-situ under totally oxygen free environment by slow addition of an acid, into Fusidic acid of a molecular dispersion form (due to the presence of a co-solvent) at the intermediate stage, and which Fusidic acid regenerates into an extremely finely dispersed form when added to a final cream base, thereby resulting in a finely and homogeneously dispersed Fusidic acid in the final cream; all operations of converting sodium fusidate into Fusidic acid carried out preferably in an environment free of atmospheric oxygen.
9. A novel dermaceutical cream as described in item 3 wherein said conversion of Sodium Fusidate into said Fusidic acid and the following formation of said Fusidic acid in a finely dispersed form in the final cream base take place in an oxygen-free environment..
10. A novel dermaceutical cream as described in item 9 wherein said oxygen-free environment comprises a gaseous environment formed of inert gas selected from a group comprising carbon dioxide, nitrogen, helium and the like.
11. A method of treating primary and secondary skin infections said method comprising applying of a cream containing Fusidic acid which is made in situ under oxygen-free environment using Sodium Fusidate, wherein said cream comprises Fusidic acid made using Sodium Fusidate, a cream base containing primary and secondary emulsifiers, waxy materials, co-solvents, acids, and water.
12. A method of treating primary and secondary skin infections said method comprising applying of a cream as described in item 11, wherein said cream further comprises any of a group comprising a buffering agent, a preservative, an anti oxidant, a chelating agent, and a humectant, or any combination thereof.
13. A method of treating primary and secondary skin infections said method comprising applying of a cream as described in item 12, wherein said Fusidic acid is present in an amount from about 0.1% (w/w) to about 25% (w/w), preferably from about 0.5% (w/w) to about 5%(w/w), and more preferably about 2.00 % (w/w), and in which the amount of Sodium Fusidate used to form in situ said Fusidic acid is in the range between about 0.1% (w/w) to about 25% (w/w), preferably from about 0.5% (w/w) to about 5% (w/w) and most preferably about 2.08 % (w/w),
   said primary and secondary emulsifier is selected from a group comprising Cetostearyl alcohol, Cetomacrogol-1000, Polysorbate-80, Span-80 and the like, either singly or any combination thereof, to form a proportion from about 1% (w/w) to 15% (w/w), preferably 15% (w/w), more preferably 14.5% (w/w),
   said waxy material is selected from a group comprising white soft paraffin, liquid paraffin, Hard paraffin and the like, either singly or any combination thereof, to form a proportion from about 5% (w/w) to 20% (w/w), preferably 15% (w/w), more preferably 12.5% (w/w),
   said co-solvent is selected from a group comprising Propylene Glycol, Hexylene Glycol, PolyEthylene Glycol-400 and the like, either singly or any combination thereof, to form a proportion from about 5% (w/w) to 40% (w/w), preferably 30% (w/w), more preferably 25% (w/w),
   said acid is selected from a group comprising HCl, H2So4, HNO3, Lactic acid and the like, either singly or any combination thereof, to form a proportion from about 0.005% (w/w) to 0.5% (w/w), preferably 0.3% (w/w), more preferably 0.25% (w/w),
   said preservative is selected from a group comprising Methylparaben, Propylparaben, Chlorocresol, Potassium sorbate, Benzoic acid and the like, either singly or any combination thereof, to form a proportion from about 0.05% (w/w) to 0.5% (w/w), preferably 0.3% (w/w), more preferably 0.2% (w/w),
   said buffering agent is selected from a group comprising Di Sodium Hydrogen Ortho Phosphate, Sodium Hydrogen Ortho Phosphate and the like, either singly or any combination thereof, to form a proportion from about 0.01% (w/w) to 1.00% (w/w), preferably 0.5% (w/w), more preferably 0.05% (w/w),
   said anti-oxidant is selected from a group comprising Butylated Hydroxy Anisole, Butylated Hydroxy Toluene and the like, either singly or any combination thereof, to form a proportion from about 0.001% (w/w) to 5% (w/w), preferably 0.1% (w/w), more preferably 0.01% (w/w),
   said chelating agent is selected from a group comprising Disodium EDTA and the like, either singly or any combination thereof, to form a proportion from about 0.01% (w/w) to 1% (w/w), preferably 0.5% (w/w), more preferably 0.1% (w/w), and
   said humectant is selected from a group comprising Glycerin, Sorbitol, Propylene glycol and the like, either singly or any combination thereof, to form a proportion from about 5% (w/w) to 40% (w/w), preferably 30% (w/w), more preferably 25% (w/w), and
   said water in the amount in the range of 20% (w/w) to 75% (w/w), preferably 35% (w/w) to 50% (w/w), more preferably 40% (w/w) to 43% (w/w), preferably purified water

It is evident from the foregoing description that the present invention has the following distinctions and advantages over the commercially available comparable products:
- It has been prepared using Sodium Fusidate which is more stable than Fusidic acid
- It has a more stable and quality enriched Fusidic acid as the final API
- The Fusidic acid in the present invention degrades more slowly than the conventional products
- The stability level of the Fusidic acid in the present invention remains within the acceptable limits throughout the shelf life of the product
- The particle size of the Fusidic acid is finer and overall particle distribution in the cream is better than the conventional products, thereby providing better dermaceutical efficacy

## Claims

1. A novel dermaceutical cream containing Fusidic acid which is made in situ under oxygen-free environment using Sodium Fusidate, wherein said cream comprises Fusidic acid made by dissolving Sodium Fusidate in a co-solvent selected from the group comprising Propylene Glycol, Hexylene Glycol, and PolyEthylene Glycol-400 in a proportion from about 5% (w/w) to 40% (w/w) under inert gas purging and under vacuum, and converting said Sodium Fusidate to Fusidic acid in-situ by adding an acid under stirring, and a cream base containing at least one of each of a primary and secondary emulsifier, a waxy material, a co-solvent, an acid, and water.

2. A novel dermaceutical cream as claimed in claim 1, wherein said Fusidic acid is present in an amount from about 0.1% (w/w) to about 25% (w/w), preferably from about 0.5% (w/w) to about 5% (w/w), and more preferably about 2.00 % (w/w), and in which the amount of said Sodium Fusidate used to form in situ said Fusidic acid is in the range between about 0.1% (w/w) to about 25% (w/w), preferably from about 0.5% (w/w) to about 5% (w/w) and more preferably about 2.08 % (w/w), and
said primary and secondary emulsifier is selected from a group comprising Cetostearyl alcohol, Cetomacrogol-1000, Polysorbate-80, and Span-80, either singly or any combination thereof, to form a proportion from about 1% (w/w) to 15% (w/w), preferably 15% (w/w), more preferably 14.5% (w/w),
said waxy material is selected from a group comprising White soft paraffin, Liquid Paraffin, and Hard paraffin, either singly or any combination thereof, to form a proportion from about 5% (w/w) to 20% (w/w), preferably 15% (w/w), more preferably 12.5% (w/w),
said co-solvent is selected from a group comprising Propylene Glycol, Hexylene Glycol, and PolyEthylene Glycol-400, either singly or any combination thereof, to form a proportion from about 5% (w/w) to 40% (w/w), preferably 30% (w/w), more preferably 25% (w/w),
said acid is selected from a group comprising acids such as HCl, H2SO4, HN03, and Lactic acid, either singly or any combination thereof, to form a proportion from about 0.005% (w/w) to 0.5% (w/w), preferably 0.3% (w/w), more preferably 0.25% (w/w), and
said water in the amount in the range of 20% (w/w) to 75% (w/w), preferably 35% (w/w) to 50% (w/w), more preferably 40% (w/w) to 43% (w/w), preferably purified water.

3. A novel dermaceutical cream as claimed in claim 2 which further comprises a preservative, wherein said preservatives is selected from a group comprising Methylparaben, Propylparaben, Chlorocresol, Potassium sorbate, and Benzoic acid, either singly or any combination thereof, to form a proportion from about 0.05% (w/w) to 0.5% (w/w), preferably 0.3% (w/w), more preferably 0.2% (w/w).

4. A novel dermaceutical cream as claimed in claim 3 which further comprises a buffering agent, wherein said buffering agent is selected from a group comprising Di Sodium Hydrogen Ortho Phosphate, and Sodium Hydrogen Ortho Phosphate, either singly or any combination thereof, to form a proportion from about 0.01 % (w/w) to 1.00% (w/w), preferably 0.5% (w/w), more preferably 0.05% (w/w).

5. A novel dermaceutical cream as claimed in claim 4 which further comprises an anti-oxidant, wherein said anti-oxidant is selected from a group comprising Butylated Hydroxy Anisole, and Butylated Hydroxy Toluene, either singly or any combination thereof, to form a proportion from about 0.001% (w/w) to 5% (w/w), preferably 0.1% (w/w), more preferably 0.01 % (w/w).

6. A novel dermaceutical cream as claimed in claim 5 which further comprises a chelating agent, wherein said chelating agent is selected from a group comprising Disodium EDTA, either singly or any combination thereof, to form a proportion from about 0.01% (w/w) to 1% (w/w), preferably 0.5% (w/w), more preferably 0.1% (w/w).

7. A novel dermaceutical cream as claimed in claim 6 which further comprises a humectant, wherein said humectant is selected from a group comprising Glycerin, Sorbitol, and Propylene glycol, either singly or any combination thereof, to form a proportion from about 5% (w/w) to 40% (w/w), preferably 30% (w/w), more preferably 25% (w/w).

8. A novel dermaceutical cream as claimed in claims 1-7 wherein sodium fusidate is converted in- situ under totally oxygen free environment by slow addition of an acid, into Fusidic acid of a molecular dispersion form (due to the presence of a co-solvent) at the intermediate stage, and which Fusidic acid regenerates into an extremely finely dispersed form when added to a final cream base, thereby resulting in a finely and homogeneously dispersed Fusidic acid in the final cream.

9. A novel dermaceutical cream as claimed in claim 8 wherein the said conversion of Sodium Fusidate into said Fusidic acid and the following formation of said Fusidic acid in a finely dispersed form in the final cream base take place in an oxygen-free environment.

10. A novel dermaceutical cream as claimed in claim 9 wherein said oxygen- free environment comprises a gaseous environment formed of inert gas selected from a group comprising carbon dioxide, nitrogen, and helium.

11. A cream containing Fusidic acid which is made in situ under oxygen-free environment by conversion of Sodium Fusidate for use in the treatment of primary and secondary skin infections, wherein said cream comprises Fusidic acid made by dissolving Sodium Fusidate in a co-solvent selected from the group comprising Propylene Glycol, Hexylene Glycol, and PolyEthylene Glycol-400 in a proportion from about 5% (w/w) to 40% (w/w) under inert gas purging and under vacuum, and converting said Sodium Fusidate to Fusidic acid in-situ by adding an acid under stirring, a cream base containing primary and secondary emulsifiers, waxy materials, co-solvents, acids, and water.

12. A cream for use in the treatment of primary and secondary skin infections as claimed in claim 11, wherein said cream further comprises any of a group comprising a buffering agent, a preservative, an anti oxidant, a chelating agent, and a humectant, or any combination thereof.

13. A cream for use in the treatment of primary and secondary skin infections as claimed in claim 12, wherein
said Sodium Fusidate used as starting material is in the range between about 0.1 % (w/w) to about 25% (w/w), preferably from about 0.5% (w/w) to about 5% (w/w) and most preferably about 2.08 % (w/w),
said primary and secondary emulsifier is selected from a group comprising Cetostearyl alcohol, Cetomacrogol-1000, Polysorbate-80, and Span-80, either singly or any combination thereof, to form a proportion from about 1% (w/w) to 15% (w/w), preferably 15% (w/w), more preferably 14.5% (w/w),
said waxy material is selected from a group comprising white soft paraffin, liquid paraffin, and Hard paraffin, either singly or any combination thereof, to form a proportion from about 5% (w/w) to 20% (w/w), preferably 15% (w/w), more preferably 12.5% (w/w),
said co-solvent is selected from a group comprising Propylene Glycol, Hexylene Glycol, and PolyEthylene Glycol-400, either singly or any combination thereof, to form a proportion from about 5% (w/w) to 40% (w/w), preferably 30% (w/w), more preferably 25% (w/w),
said acid is selected from a group comprising HCl, H2So4, HN03, and Lactic acid, either singly or any combination thereof, to form a proportion from about 0.005% (w/w) to 0.5% (w/w), preferably 0.3% (w/w), more preferably 0.25% (w/w),
said preservative is selected from a group comprising Methylparaben, Propylparaben, Chlorocresol, Potassium sorbate, and Benzoic acid, either singly or any combination thereof, to form a proportion from about 0.05% (w/w) to 0.5% (w/w), preferably 0.3% (w/w), more preferably 0.2% (w/w),
said buffering agent is selected from a group comprising Di Sodium Hydrogen Ortho Phosphate, and Sodium Hydrogen Ortho Phosphate, either singly or any combination thereof, to form a proportion from about 0.01 % (w/w) to 1.00% (w/w), preferably 0.5% (w/w), more preferably 0.05% (w/w),
said anti-oxidant is selected from a group comprising Butylated Hydroxy Anisole, and Butylated Hydroxy Toluene, either singly or any combination thereof, to form a proportion from about 0.001% (w/w) to 5% (w/w), preferably 0.1% (w/w), more preferably 0.01% (w/w),
said chelating agent is selected from a group comprising Disodium EDTA, either singly or any combination thereof, to form a proportion from about 0.01% (w/w) to 1% (w/w), preferably 0.5% (w/w), more preferably 0.1% (w/w), and
said humectant is selected from a group comprising Glycerin, Sorbitol, and Propylene glycol, either singly or any combination thereof, to form a proportion from about 5% (w/w) to 40% (w/w), preferably 30% (w/w), more preferably 25% (w/w), and
said water in the amount in the range of 20% (w/w) to 75% (w/w), preferably 35% (w/w) to 50% (w/w), more preferably 40% (w/w) to 43% (w/w), preferably purified water.

14. A process to make Fusidic acid cream, said process comprising dissolving Sodium Fusidate in a co-solvent selected from the group comprising Propylene Glycol, Hexylene Glycol, and PolyEthylene Glycol-400 to form a proportion from about 5% (w/w) to 40% (w/w) under gas purging and under vacuum, converting said Sodium Fusidate to Fusidic acid in-situ by adding an acid under stirring and adding the Fusidic acid to a cream base.

15. A process to make Fusidic acid cream as claimed in claim 14, wherein said cream base comprises a an acid, a co-solvent, an emulsifier and a waxy material along with water, preferably purified water.

## Patentansprüche

1. Neue dermazeutische Creme enthaltend Fusidinsäure, die in situ unter sauerstoff-freier Umgebung hergestellt wird unter Verwendung von Natrium-Fusidat, wobei die Creme Fusidinsäure umfasst, die durch Lösen von Natrium-Fusidat in einem Co-Lösungsmittel, das ausgewählt ist aus der Gruppe umfassend Propylen Glykol, Hexylen Glykol, und Poly-Ethylen Glykol-400 in einem Verhältnis von etwa 5% (w/w) bis 40% (w/w) unter Inertgas-Spülung und unter Vakuum hergestellt wurde, und Umsetzen des Natrium-Fusidats zu Fusidinsäure in-situ durch Zugabe einer Säure unter Rühren, und einer Cremebasis, die mindestens einen von jedem von einem primären und sekundären Emulgator enthält, ein wachsartiges Material, ein Co-Lösungsmittel, eine Säure und Wasser.

2. Neue dermazeutische Creme wie in Anspruch 1 beansprucht, wobei die Fusidinsäure in einer Menge von etwa 0,1% (w/w) bis etwa 25% (w/w) vorhanden ist, bevorzugt von etwa 0,5% (w/w) bis etwa 5% (w/w), und stärker bevorzugt etwa 2,00% (w/w), und in welcher die Menge des Natrium-Fusidats, das verwendet wird um in situ die Fusidinsäure zu bilden, im Bereich zwischen etwa 0,1% (w/w) bis etwa 25% (w/w), bevorzugt von etwa 0,5% (w/w) bis etwa 5% (w/w) und stärker bevorzugt etwa 2,08% (w/w) liegt, und
der primäre und sekundäre Emulgator ausgewählt ist aus einer Gruppe umfassend Cetostearyl-Alkohol, Cetomacrogol-1000, Polysorbat-80, und Span-80, entweder einzeln oder jede Kombination davon, um ein Verhältnis von etwa 1% (w/w) bis 15% (w/w), bevorzugt 15% (w/w), stärker bevorzugt 14,5% (w/w) zu bilden,
das wachsartige Material ausgewählt ist aus einer Gruppe umfassend, weißes weiches Paraffin, flüssiges Paraffin, und hartes Paraffin, entweder einzeln oder jede Kombination davon, um ein Verhältnis von etwa 5% (w/w) bis 20% (w/w), bevorzugt 15% (w/w), stärker bevorzugt 12,5% (w/w) zu bilden,
das Co-Lösungsmittel ausgewählt ist aus einer Gruppe umfassend Propylene Glykol, Hexylen Glykol, und PolyEthylen Glykol-400, entweder einzeln oder jede Kombination davon, um eine Verhältnis von etwa 5% (w/w) bis 40% (w/w), bevorzugt 30% (w/w), stärker bevorzugt 25% (w/w) zu bilden,
die Säure ausgewählt ist aus einer Gruppe umfassend Säuren wie HCl, H₂SO₄, HNO₃, und Milchsäure, entweder einzeln oder jede Kombination davon, um ein Verhältnis von etwa 0,005% (w/w) bis 0,5% (w/w), bevorzugt 0,3% (w/w), stärker bevorzugt 0,25% (w/w) zu bilden, und
das Wasser in der Menge in dem Bereich von 20% (w/w) bis 75% (w/w), bevorzugt 35% (w/w) bis 50% (w/w), stärker 40% (w/w) bis 43% (w/w), bevorzugt gereinigtes Wasser.

3. Neue dermazeutische Creme wie in Anspruch 2 beansprucht, die weiter ein Konservierungsmittel umfasst, wobei das Konservierungsmittel ausgewählt ist aus einer Gruppe umfassend Methylparaben, Propylparaben, Chlorocresol, Kalium-Sorbat, und Benzoesäure, entweder einzeln oder jede Kombination davon, um ein Verhältnis von etwa 0,05% (w/w) bis 0,5% (w/w), bevorzugt 0,3% (w/w), stärker bevorzugt 0,2% (w/w) zu bilden.

4. Neue dermazeutische Creme wie in Anspruch 3 beansprucht, die weiter ein pufferndes Mittel umfasst, wobei das puffernde Mittel ausgewählt ist aus einer Gruppe umfassend Di-Natrium-Hydrogen-Ortho-Phosphat, und Natrium-Hydrogen-Ortho-Phosphat, entweder einzeln oder jede Kombination davon, um ein Verhältnis von etwa 0,01% (w/w) bis 1,00% (w/w), bevorzugt 0,5% (w/w), stärker bevorzugt 0,05% (w/w) zu bilden.

5. Neue dermazeutische Creme wie in Anspruch 4 beansprucht, die weiter ein Antioxidans umfasst, wobei das Antioxidans ausgewählt ist aus einer Gruppe umfassend butyliertes Hydroxy-Anisol, und butyliertes Hydroxy-Toluol, entweder einzeln oder jede Kombination davon, um ein Verhältnis von etwa 0,001% (w/w) bis 5% (w/w), bevorzugt 0,1% (w/w), stärker bevorzugt 0,01% (w/w) zu bilden.

6. Neue dermazeutische Creme wie in Anspruch 5 beansprucht, die weiter ein chelatierendes Mittel umfasst, wobei das chelatierendes Mittel ausgewählt ist aus einer Gruppe umfassend Di-Natrium EDTA, entweder einzeln oder jede Kombination davon, um ein Verhältnis von etwa 0,01% (w/w) bis 1% (w/w), bevorzugt 0,5% (w/w), stärker bevorzugt 0,1% (w/w) zu bilden.

7. Neue dermazeutische Creme wie in Anspruch 6 beansprucht, die weiter ein Befeuchtungsmittel umfasst, wobei das Befeuchtungsmittel ausgewählt ist aus einer Gruppe umfassend Glycerin, Sorbitol, und Propylen Glykol, entweder einzeln oder jede Kombination davon , um ein Verhältnis von etwa 5% (w/w) bis 40% (w/w), bevorzugt 30% (w/w), stärker bevorzugt 25% (w/w) zu bilden.

8. Neue dermazeutische Creme wie in Ansprüchen 1-7 beansprucht, wobei Natrium-Fusidat in-situ unter vollkommen sauerstoff-freier Umgebung durch langsame Zugabe einer Säure, in Fusidinsäure einer molekularen Dispersionsform (auf Grund der Anwesenheit eines Co-Lösungsmittels) bei dem Intermediat-Stadium umgesetzt wird, und die Fusidinsäure in eine extrem fein dispergierte Form regeneriert, wenn sie zu einer finale Cremebasis zugegeben wird, wobei dadurch eine fein und homogen dispergierte Fusidinsäure in der finalen Creme entsteht.

9. Neue dermazeutische Creme wie in Anspruch 8 beansprucht, wobei die Umsetzen von Natrium-Fusidat in Fusidinsäure und die folgende Bildung der Fusidinsäure in einer fein dispergierten Form in der finalen Cremebasis finden in einer sauerstofffreien Umgebung statt.

10. Neue dermazeutische Creme wie in Anspruch 9 beansprucht, wobei die sauerstofffreie Umgebung eine gasartige Umgebung umfasst, die aus inertem Gas gebildet wird, das ausgewählt ist aus einer Gruppe umfassend Kohlendioxid, Stickstoff und Helium.

11. Creme enthaltend Fusidinsäure, die in situ unter sauerstoff-freier Umgebung durch Umsetzen von Natrium-Fusidat hergestellt wird, für die Verwendung zur Behandlung von primären und sekundären Hautinfektionen, wobei die Creme Fusidinsäure umfasst, die durch Lösen von Natrium-Fusidat in einem Co-Lösungsmittel hergestellt wurde, das ausgewählt ist aus der Gruppe bestehend aus Propylen Glykol, Hexylen Glykol und PolyEthylen Glykol-400 in einem Verhältnis von etwa 5% (w/w) bis 40% (w/w) unter Inertgas-Spülung und unter Vakuum, und Umsetzen des Natrium-Fusidat zu Fusidinsäure in-situ durch Zugabe von Säure unter Rühren, eine Cremebasis, die primäre und sekundäre Emulgatoren enthält, wachsartiges Material, Co-Lösungsmittel, Säuren, und Wasser.

12. Creme für die Verwendung in der Behandlung von primären und sekundären Hautinfektionen wie in Anspruch 11 beansprucht, wobei die Creme weiter jedes einer Gruppe umfasst, die ein pufferndes Mittel, ein Konservierungsmittel, ein Antioxidans, ein chelatierendes Mittel, und ein Befeuchtungsmittel, oder jede Kombination davon umfasst.

13. Creme zur Verwendung in der Behandlung von primären und sekundären Hautinfektionen wie in Anspruch 12 beansprucht, wobei
das Natrium-Fusidat, das als Ausgangsmaterial verwendet wird, in einem Bereich zwischen etwa 0,1% (w/w) bis etwa 25% (w/w), bevorzugt von etwa 0,5% (w/w) bis etwa 5% (w/w) und am stärksten bevorzugt etwa 2,08% (w/w) ist,
der primäre und sekundäre Emulgator ausgewählt ist aus einer Gruppe umfassend Cetostearyl Alkohol, Cetomacrogol-1000, Polysorbat-80, und Span-80, entweder einzeln oder in Kombination davon, um ein Verhältnis von etwa 1% (w/w) bis 15% (w/w), bevorzugt 15% (w/w), stärker bevorzugt 14,5% (w/w) zu bilden,
das wachsartige Material ausgewählt ist aus einer Gruppe umfassend weißes weiches Paraffin, flüssiges Paraffin, und hartes Paraffin, entweder einzeln oder jede Kombination davon, um ein Verhältnis von etwa 5% (w/w) bis 20% (w/w), bevorzugt 15% (w/w), stärker bevorzugt 12,5% (w/w) zu bilden,
das Co-Lösungsmittel ausgewählt ist aus einer Gruppe umfassend Propylen Glykol, Hexylen Glykol und PolyEthylen Glykol-400, entweder einzeln oder jede Kombination davon, um ein Verhältnis von etwa 5% (w/w) bis 40% (w/w), bevorzugt 30% (w/w), stärker bevorzugt 25% (w/w) zu bilden,
die Säure ausgewählt ist aus einer Gruppe umfassend Säuren wie HCl, H₂SO₄, HNO₃, und Milchsäure, entweder einzeln oder jede Kombination davon, um ein Verhältnis von etwa 0,005% (w/w) bis 0,5% (w/w), bevorzugt 0,3% (w/w), stärker bevorzugt 0,25% (w/w) zu bilden,
das Konservierungsmittel ausgewählt ist aus einer Gruppe umfassend Methylparaben, Propylparaben, Chlorocresol, Kalium-Sorbat, und Benzoesäure, entweder einzeln oder jede Kombination davon, um ein Verhältnis von etwa 0,05% (w/w) bis 0,5% (w/w), bevorzugt 0,3% (w/w), stärker bevorzugt 0,2% (w/w) zu bilden,
das puffernde Mittel ausgewählt ist aus einer Gruppe umfassend Di-Natrium-Hydrogen-Ortho-Phosphat, und Natrium-Hydrogen-Ortho-Phosphat, entweder einzeln oder jede Kombination davon, um ein Verhältnis von etwa 0,01% (w/w) bis 1,00% (w/w), bevorzugt 0,5% (w/w), stärker bevorzugt 0,05% (w/w) zu bilden,
das Antioxidans ausgewählt ist aus einer Gruppe umfassend butyliertes Hydroxy-Anisol, und butyliertes Hydroxy-Toluol, entweder einzeln oder jede Kombination davon, um ein Verhältnis von etwa 0,001% (w/w) bis 5% (w/w), bevorzugt 0,1% (w/w), stärker bevorzugt 0,01% (w/w) zu bilden,
das chelatierendes Mittel ausgewählt ist aus einer Gruppe umfassend Di-Natrium EDTA, entweder einzeln oder jede Kombination davon, um ein Verhältnis von etwa 0,01% (w/w) bis 1% (w/w), bevorzugt 0,5% (w/w), stärker bevorzugt 0,1% (w/w) zu bilden, und
das Befeuchtungsmittel ausgewählt ist aus einer Gruppe umfassend Glycerin, Sorbitol, und Propylene Glykol, entweder einzeln oder jede Kombination davon, um ein Verhältnis von etwa 5% (w/w) bis 40% (w/w), bevorzugt 30% (w/w), stärker bevorzugt 25% (w/w) zu bilden, und
das Wasser in der Menge in dem Bereich von 20% (w/w) bis 75% (w/w), bevorzugt 35% (w/w) bis 50% (w/w), stärker bevorzugt 40% (w/w) bis 43% (w/w), bevorzugt gereinigtes Wasser.

14. Verfahren zur Herstellung von Fusidinsäure-Creme, wobei das Verfahren Lösen von Natrium-Fusidat in einem Co-Lösungsmittel umfasst, das ausgewählt ist aus der Gruppe bestehend aus Propylen Glykol, Hexylen Glykol, und Poly-Ethylen Glykol-400 um ein Verhältnis von etwa 5% (w/w) bis 40% (w/w) zu bilden unter Inertgas-Spülung und unter Vakuum, Umsetzen des Natrium-Fusidats zu Fusidinsäure in-situ durch Zugabe einer Säure unter Rühren und Zugabe der Fusidinsäure zu einer Cremebasis.

15. Verfahren zur Herstellung von Fusidinsäure-Creme wie in Anspruch 14 beansprucht, wobei die Cremebasis eine Säure, ein Co-Lösungsmittel, einen Emulgator und ein wachsartiges Material zusammen mit Wasser, bevorzugt gereinigtes Wasser umfasst.

## Revendications

1. Crème dermaceutique nouvelle contenant de l'acide fusidique qui est préparé in situ dans un environnement dépourvu d'oxygène en utilisant du fusidate de sodium, ladite crème comprenant de l'acide fusidique préparé en dissolvant du fusidate de sodium dans un cosolvant choisi dans le groupe comprenant le propylèneglycol, l'hexylèneglycol et le Polyéthylèneglycol 400 en une proportion d'environ 5 % (en poids/poids) à 40 % (en poids/poids) dans des conditions de purge avec un gaz inerte et sous vide, et en convertissant ledit fusidate de sodium en acide fusidique in situ par addition d'un acide sous agitation, et un excipient pour crèmes contenant au moins un de chacun d'un émulsionnant primaire et d'un émulsionnant secondaire, d'une matière cireuse, d'un cosolvant, d'un acide, et de l'eau.

2. Crème dermaceutique nouvelle suivant la revendication 1, dans laquelle ledit acide fusidique est présent en une quantité d'environ 0,1 % (en poids/poids) à environ 25 % (en poids/poids), avantageusement d'environ 0,5 % (en poids/poids) à environ 5 % (en poids/poids) et plus avantageusement d'environ 2,00 % (en poids/poids), et dans laquelle la quantité dudit fusidate de sodium utilisé pour former in situ ledit acide fusidique est comprise dans l'intervalle entre environ 0,1 % (en poids/poids) et environ 25 % (en poids/poids), avantageusement d'environ 0,5 % (en poids/poids) à environ 5 % (en poids/poids) et est plus avantageusement d'environ 2,08 % (en poids/poids), et
lesdits émulsionnants primaire et secondaire sont choisis dans un groupe comprenant l'alcool stéarylique, le Cétomacrogol 1000, le Polysorbate 80 et le Span 80, isolément ou sous forme de n'importe laquelle de leurs associations, pour parvenir à une proportion d'environ 1 % (en poids/poids) à 15 % (en poids/poids), avantageusement de 15 % (en poids/poids), plus avantageusement de 14,5 % (en poids/poids),
ladite matière cireuse est choisie dans un groupe comprenant la paraffine molle blanche, la paraffine liquide et la paraffine dure, isolément ou sous forme de n'importe laquelle de leurs associations, pour parvenir à une proportion d'environ 5 % (en poids/poids) à 20 % (en poids/poids), avantageusement de 15 % (en poids/poids), plus avantageusement de 12,5 % (en poids/poids),
ledit cosolvant est choisi dans un groupe comprenant le propylèneglycol, l'hexylèneglycol et le Polyéthylèneglycol 400, isolément ou sous forme de n'importe laquelle de leurs associations, pour parvenir à une proportion d'environ 5 % (en poids/poids) à 40 % (en poids/poids), avantageusement de 30 % (en poids/poids), plus avantageusement de 25 % (en poids/poids),
ledit acide est choisi dans un groupe comprenant des acides tels que HCl, H₂SO₄, HNO₃ et l'acide lactique, isolément ou sous forme de n'importe laquelle de leurs associations, pour parvenir à une proportion d'environ 0,005 % (en poids/poids) à 0,5 % (en poids/poids), avantageusement de 0,3 % (en poids/poids), plus avantageusement de 0,25 % (en poids/poids), et
ladite eau en une quantité dans l'intervalle de 20 % (en poids/poids) à 75 % (en poids/poids), avantageusement de 35 % (en poids/poids) à 50 % (en poids/poids), plus avantageusement de 40 % (en poids/poids) à 43 % (en poids/ poids), de préférence de l'eau purifiée.

3. Crème dermaceutique nouvelle suivant la revendication 2, qui comprend en outre un conservateur, dans laquelle ledit conservateur est choisi dans un groupe comprenant le parahydroxybenzoate de méthyle, le parahydroxybenzoate de propyle, le chlorocrésol, le sorbate de potassium et l'acide benzoïque, isolément ou sous forme de n'importe laquelle de leurs associations, pour parvenir à une proportion d'environ 0,05 % (en poids/poids) à 0,5 % (en poids/poids), avantageusement de 0,3 % (en poids/poids), plus avantageusement de 0,2 % (en poids/poids).

4. Crème dermaceutique nouvelle suivant la revendication 3, qui comprend en outre un tampon, dans laquelle ledit tampon est choisi dans un groupe comprenant l'orthophosphate disodique et l'orthophosphate monosodique, isolément ou sous forme de n'importe laquelle de leurs associations, pour parvenir à une proportion d'environ 0,01 % (en poids/poids) à 1,00 % (en poids/poids), avantageusement de 0,5 % (en poids/poids), plus avantageusement de 0,05 % (en poids/poids).

5. Crème dermaceutique nouvelle suivant la revendication 4, qui comprend en outre un antioxydant, dans laquelle ledit antioxydant est choisi dans un groupe comprenant le butylhydroxy-anisole et le butylhydroxy-toluène, isolément ou sous forme de n'importe laquelle de leurs associations, pour parvenir à une proportion d'environ 0,001 % (en poids/poids) à 5 % (en poids/poids), avantageusement de 0,1 % (en poids/poids), plus avantageusement de 0,01 % (en poids/poids).

6. Crème dermaceutique nouvelle suivant la revendication 5, qui comprend en outre un agent chélatant, dans laquelle ledit agent chélatant est choisi dans un groupe comprenant l'EDTA disodique, isolément ou sous forme de n'importe quelle association, pour parvenir à une proportion d'environ 0,01 % (en poids/poids) à 1 % (en poids/poids), avantageusement de 0,5 % (en poids/poids), plus avantageusement de 0,1 % (en poids/poids).

7. Crème dermaceutique nouvelle suivant la revendication 6, qui comprend en outre un agent humidifiant, dans laquelle ledit agent humidifiant est choisi dans un groupe comprenant le glycérol, le sorbitol et le propylèneglycol, isolément ou sous forme de n'importe laquelle de leurs associations, pour parvenir à une proportion d'environ 5 % (en poids/poids) à 40 % (en poids/poids), avantageusement de 30 % (en poids/poids), plus avantageusement de 25 % (en poids/poids).

8. Crème dermaceutique nouvelle suivant les revendications 1 à 7, dans laquelle le fusidate de sodium est converti in situ dans un environnement totalement dépourvu d'oxygène par addition lente d'un acide, en acide fusidique sous forme d'une dispersion moléculaire (en raison de la présence d'un cosolvant) au stade intermédiaire, acide fusidique qui se régénère sous une forme extrêmement finement dispersée lors de l'addition à un excipient pour crème finale, avec ainsi pour résultat un acide fusidique dispersé finement et de manière homogène dans la crème finale.

9. Crème dermaceutique nouvelle suivant la revendication 8, dans laquelle ladite conversion du fusidate de sodium en ledit acide fusidique et la formation dudit acide fusidique sous une forme finement dispersée dans l'excipient pour crème finale s'effectuent dans un environnement dépourvu d'oxygène.

10. Crème dermaceutique nouvelle suivant la revendication 9, dans laquelle ledit environnement dépourvu d'oxygène comprend un environnement gazeux formé d'un gaz inerte choisi dans un groupe comprenant le dioxyde de carbone, l'azote et l'hélium.

11. Crème contenant de l'acide fusidique qui est préparé in situ dans un environnement dépourvu d'oxygène par conversion de fusidate de sodium pour une utilisation dans le traitement d'infections cutanées primaires et secondaires, ladite crème comprenant de l'acide fusidique préparé en dissolvant du fusidate de sodium dans un cosolvant choisi dans le groupe comprenant le propylèneglycol, l'hexylèneglycol et le Polyéthylèneglycol 400 en une proportion d'environ 5 % (en poids/poids) à 40 % (en poids/poids) dans des conditions de purge avec un gaz inerte et sous vide, et en convertissant ledit fusidate de sodium en acide fusidique in situ par addition d'un acide sous agitation, un excipient pour crèmes contenant des émulsionnants primaire et secondaire, des matières cireuses, des cosolvants, des acides, et de l'eau.

12. Crème pour une utilisation dans le traitement d'infections cutanées primaires et secondaires suivant la revendication 11, ladite crème comprenant en outre n'importe quel agent d'un groupe comprenant un tampon, un conservateur, un antioxydant, un agent chélatant et un agent humidifiant, ou n'importe laquelle de leurs associations.

13. Crème pour une utilisation dans le traitement d'infections cutanées primaires et secondaires suivant la revendication 12, dans laquelle
ledit fusidate de sodium utilisé comme matière de départ est présent en une quantité dans l'intervalle entre environ 0,1 % (en poids/poids) et environ 25 % (en poids/poids), avantageusement d'environ 0,5 % (en poids/poids) à environ 5 % (en poids/poids) et de préférence égale à environ 2,08 % (en poids/poids),
lesdits émulsionnants primaire et secondaire sont choisis dans un groupe comprenant l'alcool cétostéarylique, le Cétomacrogol 1000, le Polysorbate 80 et le Span 80, isolément ou sous forme de n'importe de laquelle de leurs associations, pour parvenir à une proportion d'environ 1 % (en poids/poids) à 15 % (en poids/poids), avantageusement de 15 % (en poids/poids), plus avantageusement de 14,5 % (en poids/poids),
ladite matière cireuse est choisie dans un groupe comprenant la paraffine molle blanche, la paraffine liquide et la paraffine dure, isolément ou sous forme de n'importe laquelle de leurs associations, pour parvenir à une proportion d'environ 5 % (en poids/poids) à 20 % (en poids/poids), avantageusement de 15 % (en poids/poids), plus avantageusement de 12,5 % (en poids/poids),
ledit cosolvant est choisi dans un groupe comprenant le propylèneglycol, l'hexylèneglycol et le Polyéthylèneglycol 400, isolément ou sous forme de n'importe laquelle de leurs associations, pour parvenir à une proportion d'environ 5 % (en poids/poids) à 40 % (en poids/poids), avantageusement de 30 % (en poids/poids), plus avantageusement de 25 % (en poids/poids),
ledit acide est choisi dans un groupe comprenant des acides tels que HCl, H₂SO₄, HNO₃ et l'acide lactique, isolément ou sous forme de n'importe laquelle de leurs associations, pour parvenir à une proportion d'environ 0,005 % (en poids/poids) à 0,5 % (en poids/poids), avantageusement de 0,3 % (en poids/poids), plus avantageusement de 0,25 % (en poids/poids),
ledit conservateur est choisi dans un groupe comprenant le parahydroxybenzoate de méthyle, le parahydroxybenzoate de propyle, le chlorocrésol, le sorbate de potassium et l'acide benzoïque, isolément ou sous forme de n'importe laquelle de leurs associations, pour parvenir à une proportion d'environ 0,05 % (en poids/poids) à 0,5 % (en poids/poids), avantageusement de 0,3 % (en poids/poids), plus avantageusement de 0,2 % (en poids/poids),
ledit tampon est choisi dans un groupe comprenant l'orthophosphate disodique et l'orthophosphate monosodique, isolément ou sous forme de n'importe laquelle de leurs associations, pour parvenir à une proportion d'environ 0,01 % (en poids/poids) à 1,00 % (en poids/poids), avantageusement de 0,5 % (en poids/poids), plus avantageusement de 0,05 % (en poids/poids),
ledit antioxydant est choisi dans un groupe comprenant le butylhydroxy-anisole et le butylhydroxy-toluène, isolément ou sous forme de n'importe laquelle de leurs associations, pour parvenir à une proportion d'environ 0,001 % (en poids/poids) à 5 % (en poids/poids), avantageusement de 0,1 % (en poids/poids), plus avantageusement de 0,01 % (en poids/poids),
ledit agent chélatant est choisi dans un groupe comprenant l'EDTA disodique, isolément ou sous forme de n'importe quelle association, pour parvenir à une proportion d'environ 0,01 % (en poids/poids) à 1 % (en poids/poids), avantageusement de 0,5 % (en poids/poids), plus avantageusement de 0,1 % (en poids/poids), et
ledit agent humidifiant est choisi dans un groupe comprenant le glycérol, le sorbitol et le propylèneglycol, isolément ou sous forme de n'importe laquelle de leurs associations, pour parvenir à une proportion d'environ 5 % (en poids/poids) à 40 % (en poids/poids), avantageusement de 30 % (en poids/poids), plus avantageusement de 25 % (en poids/poids), et
ladite eau en une quantité dans l'intervalle de 20 % (en poids/poids) à 75 % (en poids/poids), avantageusement de 35 % (en poids/poids) à 50 % (en poids/poids), plus avantageusement de 40 % (en poids/poids) à 43 % (en poids/ poids), de préférence de l'eau purifiée.

14. Procédé pour la préparation d'une crème à l'acide fusidique, ledit procédé comprenant la dissolution de fusidate de sodium dans un cosolvant choisi dans le groupe comprenant le propylèneglycol, l'hexylèneglycol et le Polyéthylèneglycol 400, pour parvenir à une proportion d'environ 5 % (en poids/poids) à 40 % (en poids/poids) dans des conditions de purge avec un gaz inerte et sous vide, la conversion dudit fusidate de sodium en acide fusidique in situ en ajoutant un acide sous agitation et l'addition de l'acide fusidique à un excipient pour crèmes.

15. Procédé pour la préparation d'une crème à l'acide fusidique suivant la revendication 14, dans lequel ledit excipient pour crèmes comprend un acide, un cosolvant, un émulsionnant et une matière cireuse avec de l'eau, de préférence de l'eau purifiée.
